# FASCICULE DE BREVET EUROPEEN

(11) **EP 1 230 222 B1**
(45) Date de publication et mention de la délivrance du brevet: **07.07.2004**
(21) Numéro de dépôt: 00974627.2
(22) Date de dépôt: 02.11.2000
(51) Int. Cl.: C07D 231/54, A61K 31/416, A61P 29/00, A61P 37/00

(54) **DERIVES TRICYCLIQUES D'ACIDES 1-BENZYLPYRAZOLE-3-CARBOXYLIQUE COMME ANTAGONISTES DES RECEPTEURS DE CANNABINOIDES**
TRIZYKLISCHE 1-BENZYLPYRAZOL-3-CARBONSÄUREDERIVATE ALS CANNABINOIDREZEPTORANTAGONISTEN
1-BENZYLPYRAZOLES-3-CARBOXYLIC ACID TRICYCLIC DERIVATIVES AS CANNABINOID RECEPTOR ANTAGONISTS

(30) Priorité: 03.11.1999 FR 9913847
(43) Date de publication de la demande: 14.08.2002
(73) Titulaire: SANOFI-SYNTHELABO, 75013 Paris (FR)
(72) Inventeur: BARTH, Francis, F-34680 Saint-Georges-d'Orques (FR); MILLAN, Joseph, F-34990 Juvignac (FR); OUSTRIC, Didier, F-34920 Le Crès (FR); RINALDI, Murielle, F-34680 Saint-Georges-d'Orques (FR); VERNHET, Martine, F-34000 Montpellier (FR)
(74) Mandataire: Thouret-Lemaitre, Elisabeth
(86) Numéro de dépôt international: PCT/FR2000/003047
(87) Numéro de publication internationale: WO 2001/032629

(56) Documents cités:
- EP-A- 0 656 354
- WO-A-96/09304
- GB-A- 1 382 773
- US-A- 5 696 143
- US-A- 5 925 768

## Description

La présente invention a pour objet des composés antagonistes des récepteurs aux cannabinoïdes CB₂, leur préparation, les compositions pharmaceutiques en contenant. Les composés de l'invention sont des dérivés tricycliques d'acide 1-benzylpyrazole-3-carboxylique.

Les demandes de brevet EP-A-576 357, EP-A-658 546 et WO-97/19063 décrivent des dérivés du pyrazole présentant une affinité pour les récepteurs aux cannabinoïdes. Plus particulièrement, la demande de brevet EP-A-656 354 revendique le N-pipéridino-5-(4-chlorophényl)-1-(2,4-dichlorophényl)-4-méthylpyrazole-3-carboxamide et ses sels pharmaceutiquement acceptables qui présentent une très bonne affinité pour les récepteurs aux cannabinoïdes CB₁. La demande de brevet EP-A-868 420 décrit des dérivés de pyrazole-3-carboxamide substitué en 1 du pyrazole par un groupe benzyle diversement substitué.

La demande de brevet internationale WO-96/09304 décrit des composés inhibant la cyclooxygénase, plus spécifiquement la cyclooxygénase-2. Ces composés utiles dans le traitement de l'inflammation et des maladies inflammatoires répondent à la formule : 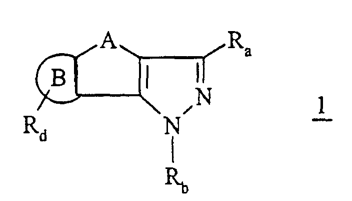 dans laquelle :
A, B, Rₐ, R_{b}, R_{d} ont différentes significations.

On a maintenant trouvé des nouveaux dérivés tricycliques d'acide 1-benzylpyrazole-3-carboxylique qui possèdent une très bonne affinité pour les récepteurs CB₂ des cannabinoïdes et sont utiles dans les domaines thérapeutiques où le cannabis est connu pour intervenir.

Le Δ⁹-THC est le principal constituant actif extrait de *Cannabis sativa* (Tuner, 1985 ; In Marijuana 84, Ed. Harvey, DY, IRL Press, Oxford).

Les effets des cannabinoïdes sont dûs à une intéraction avec des récepteurs spécifiques de haute affinité présents au niveau central (Devane et al., Mol. Pharmacol., 1988, 34, 605-613) et périphérique (Nye et al., Pharmacol. and Experimental Ther., 1985, 234, 784-791 ; Kaminski et al., 1992, Mol. Pharmacol., 42, 736-742 ; Munro et al., Nature, 1993, 365, 61-65).

La caractérisation des récepteurs a été rendue possible par la mise au point de ligands synthétiques des récepteurs aux cannabinoïdes tels que les agonistes WIN 55212-2 (J. Pharmacol. Exp. Ther., 1993, 264, 1352-1363) ou le CP 55,940 (J. Pharmacol. Exp. Ther., 1988, 247, 1046-1051).

La présente invention a pour objet des composés de formule : 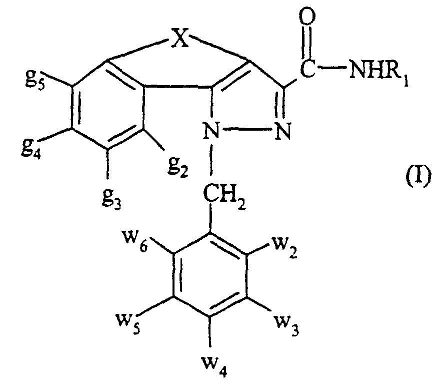 dans laquelle :
- X- représente un groupe -(CH₂)ₙ- ;
- n est égal à 1 ou 2 ;
- g₂, g₃, g₄, g₅, w₂, w₃, w₄, w₅, w₆ sont identiques ou différents et représentent chacun indépendamment l'hydrogène, un halogène, un trifluorométhyle, un (C₁-C₄)alkyle, un (C₁-C₄)alcoxy, un (C₁-C₄)alkylthio, un nitro ;
- R₁ représente un radical carbocyclique non aromatique en C₃-C₁₅ non substitué ou substitué une ou plusieurs fois par un (C₁-C₄)alkyle ;
ainsi que leurs sels et leurs solvats.

Par alkyle, on entend des alkyles droits ou ramifiés. Les groupes méthyle, éthyle, propyle, isopropyle étant préférés.

Par radical carbocyclique non aromatique en C₃-C₁₅, on entend un radical saturé, mono ou polycyclique, condensé ou ponté. Ces radicaux comprennent en particulier les radicaux suivants : cyclopentyle, cyclohexyle, adamantyle, bicyclo[3.2.1]octyle, ainsi que le 1,3,3-triméthylbicyclo[2.2.1]heptyle ou fenchyle, le 7,7-diméthylbicyclo [4.1.0]hept-3-yle.

Par halogène, on entend un atome de chlore, de brome, de fluor ou d'iode.

Les sels éventuels des composés de formule (I) comprennent les sels d'addition d'acide pharmaceutiquement acceptables tels que le chlorhydrate, le bromhydrate, le sulfate, l'hydrogénosulfate, le dihydrogénophosphate, le méthanesulfonate, le méthylsulfate, le maléate, l'oxalate, le fumarate, le naphtalènesulfonate, le glyconate, le gluconate, le citrate, l'iséthionate, le paratoluènesulfonate, le méthylènesulfonate, le benzènesulfonate ou le succinate.

La présente invention a tout particulièrement pour objet des composés de formule: 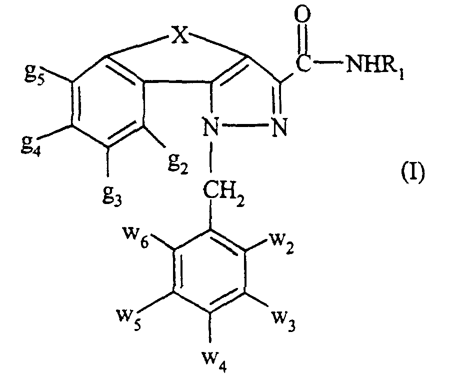 dans laquelle :
- X- représente un groupe -(CH₂)ₙ- ;
- n est égal à 1 ou 2 ;
- g₂, g₃, g₄, g₅, w₂, w₃, w₄, w₅, w₆ sont identiques ou différents et représentent chacun indépendamment l'hydrogène, un halogène, un trifluorométhyle, un (C₁-C₃)alk_{Y}le, un (C₁-C₃)alcoxy, un ((C₁-C₃)alkylthio, un nitro ;
- R₁ représente un radical carbocyclique non aromatique en C₃-C₁₅ non substitué ou substitué une ou plusieurs fois par un (C₁-C₄)alkyle ;
ainsi que leurs sels et leurs solvats.

Parmi les composés de formule (I), on préfère ceux dans lesquels g₂, g₅, w₅, w₆ représentent l'hydrogène et g₃, g₄, w₂, w₃ et w₄ ont l'une des valeurs définies ci-dessus pour les composés de formule (I) hormis l'hydrogène. Plus particulièrement, on préfère les composés de formule (I) dans lesquels w₂, w₃ et w₄ représentent le chlore ou un méthyle et g₃ et g₄ représente le chlore, le brome ou un méthyle, les autres substituants w et g étant l'hydrogène.

On préfère également les composés de formule (I) dans laquelle R₁ représente un radical carbocyclique choisi parmi : le 1,3,3-triméthylbicyclo[2.2.1]hept-2-yle, le bicyclo[3.2.1]oct-3-yle, le 7,7-diméthylbicyclo[4.1.0]hept-3-yle.

Parmi les composés de formule (I) on distingue ceux dans lesquels -X- représente un groupe -CH₂-CH₂-, et ceux dans lesquels -X- représente un groupe -CH₂-. Les composés de formule (I) dans laquelle -X- représente -CH₂- sont préférés.

Dans la présente description, on utilise les abréviations suivantes :
éther : éther diéthylique
éther iso : éther diisopropylique
EtOH : éthanol
MeOH : méthanol
DCM : dichlorométhane
AcOEt : acétate d'éthyle
LiHMDS : sel de lithium de l'hexaméthyldisilazane
(CO₂Et)₂ : oxalate d'éthyle
APTS : acide paratoluènesulfonique
PPA : acide polyphosphorique
DIBAL : diisobutyl aluminium hydrure
AcOH : acide acétique
TA : température ambiante
F : point de fusion
Eb : point d'ébullition
p : pression
RMN : résonnance magnétique nucléaire. Les spectres RMN sont enregistrés à 200 MHz dans le DMSO d6
s: singulet ; d : doublet ; t : triplet ; m : massif ou multiplet.

La présente invention a également pour objet un procédé de préparation d'un composé selon l'invention, de ses sels et de ses solvats. Ce procédé est caractérisé en ce que l'on traite un dérivé fonctionnel d'un acide de formule : 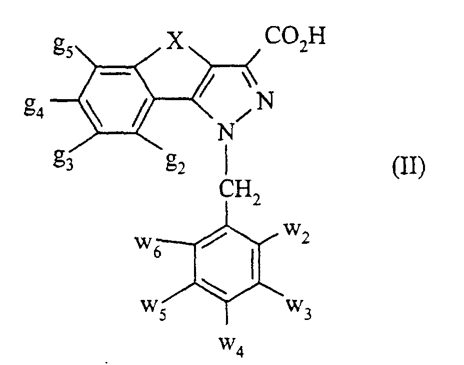 dans laquelle -X- et g₂, g₃, g₄, g₅, w₂, w₃, w₄, w₅, w₆ sont tels que définis ci-dessus pour (I), avec un composé de formule NH₂R₁ (III), dans laquelle R₁ est tel que défini ci-dessus pour (I).

La réaction est effectuée en milieu basique, par exemple en présence de triéthylamine dans un solvant tel que le dichlorométhane ou le tétrahydrofurane.

Comme dérivé fonctionnel de l'acide (II) on peut utiliser le chlorure d'acide, l'anhydride, un anhydride mixte, un ester alkylique en C₁-C₄ dans lequel l'alkyle est droit ou ramifié, un ester activé, par exemple l'ester de *p*-nitrophényle, ou l'acide libre opportunément activé, par exemple, avec le N,N-dicyclohexylcarbodiimide ou avec l'hexafluorophosphate de benzotriazol-N-oxotris(diméthylamino)phosphonium (BOP).

Ainsi par le procédé selon l'invention, on peut faire réagir le chlorure de l'acide de formule (II) obtenu par réaction du chlorure de thionyle sur l'acide de formule (II) dans un solvant inerte tel que le benzène ou le toluène ou un solvant chloré (le dichlorométhane, le dichloroéthane, le chloroforme par exemple), un éther (tétrahydrofurane, dioxane par exemple) ou un amide (N,N-diméthylformamide par exemple) sous une atmosphère inerte, à une température comprise entre 0°C et la température de reflux du solvant.

Une variante au mode opératoire consiste à préprarer l'anhydride mixte de l'acide de formule (II) par réaction du chloroformiate d'éthyle avec l'acide de formule (II), en présence d'une base telle que la triéthylamine.

L'acide de départ (II) est nouveau et constitue un autre aspect de la présente invention, ses dérivés fonctionnels sont également nouveaux, notamment son chlorure d'acide et son ester alkylique en C₁-C₄.

L'acide de formule (II) peut être obtenu en suivant le schéma réactionnel ci-après :

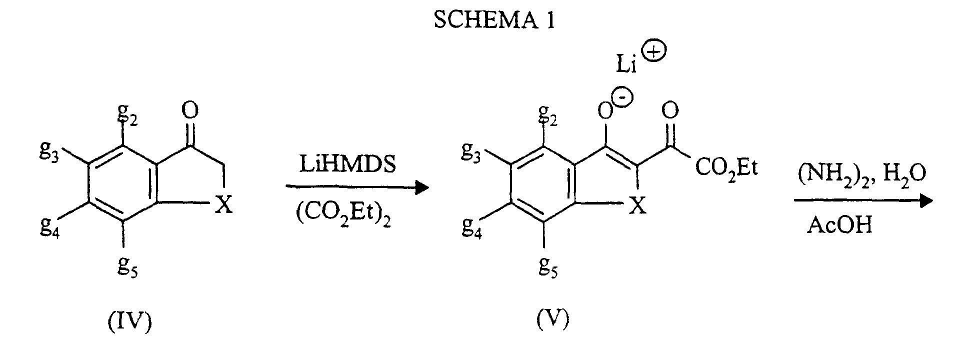

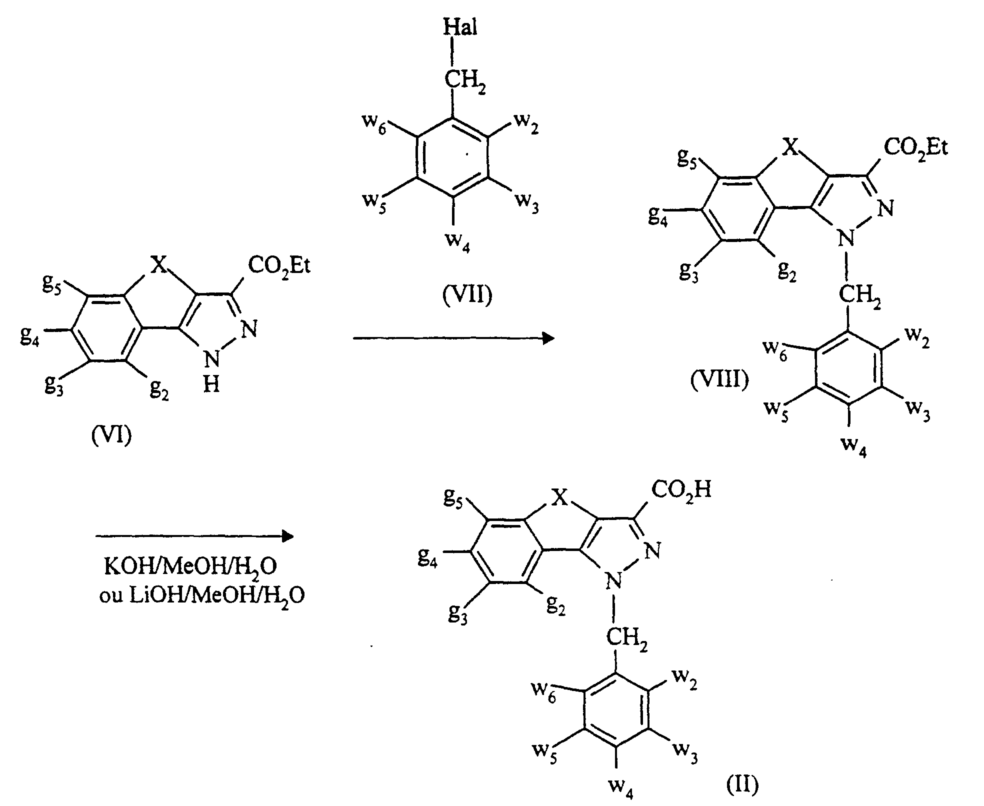

L'acide de formule (II) peut également être préparé par action d'un dérivé de la benzylhydrazine de formule (IX) sur un composé de formule (V), selon le SCHEMA 2 ci-après.

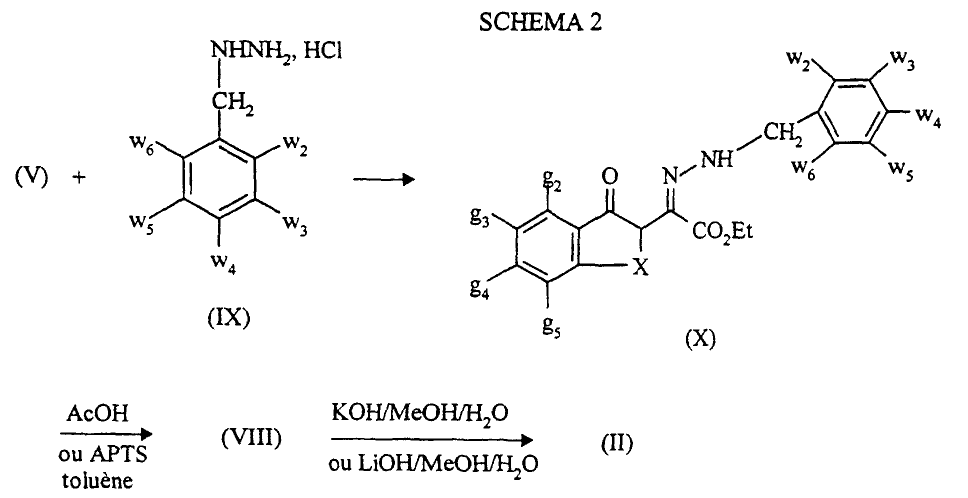

En utilisant le procédé selon le SCHEMA 1, par action du dérivé d'halogènure de benzyle de formule (VII) sur le composé de formule (VI), il peut également se former un isomère de position du composé de formule (VIII), à savoir un composé de formule

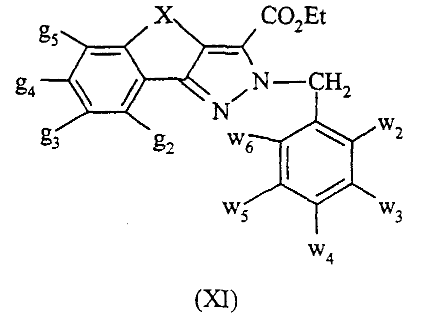

De même, en utilisant le procédé selon le SCHEMA 2, par action du dérivé de benzylhydrazine de formule (IX) dans laquelle w₂ à w₆ sont tels que définis ci-dessus pour (I), il peut également se former un isomère de position du composé de formule (VIII), à savoir un composé de formule (XI).

Pour obtenir l'acide de formule (II), on peut soit séparer les 2 isomères de formule (VIII) et (XI), soit effectuer l'hydrolyse du mélange des isomères (VIII) et (XI) pour préparer un mélange de l'acide de formule (II) et de son isomère de formule :

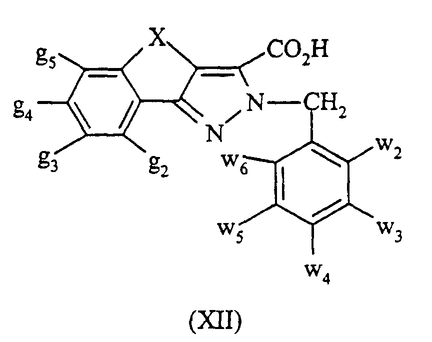

La séparation des isomères est effectuée par des méthodes classiques, par exemple par chromatographie ou par cristallisation.

Les composés de formule (IV) sont connus ou préparés par des méthodes connues. Par exemple, les tétralones de formule (IV) dans laquelle -X- représente -CH₂-CH₂- sont connues ou préparées par des méthodes connues telles que décrites dans Synthetic Communications, 1991, 21, 981-987.

On prépare le sel de lithium de formule (V) par action du sel de lithium de l'hexaméthyldisilazane puis de l'oxalate d'éthyle.

Par action de l'hydrate d'hydrazine, puis chauffage en présence d'acide acétique ou en présence d'acide paratoluènesulfonique dans le toluène, on prépare le composé de formule (VI). On traite ensuite le composé de formule (VI) par une base forte telle que l'hydrure de sodium ou l'amidure de sodium dans un solvant puis on fait agir un halogénure de benzyle de formule (VII) dans laquelle Hal représente un halogène, de préférence le chlore ou le brome et w₂-w₆ sont tels que définis ci-dessus pour (I). On effectue ensuite une saponification selon les méthodes classiques, par exemple en présence de potasse ou d'hydroxyde de lithium dans le méthanol, pour obtenir l'acide attendu de formule (II).

Les halogénures de benzyle de formule (VII) sont connus ou préparés par des méthodes connues.

D'une manière générale, les composés de formule (VII) dans laquelle Hal représente un atome de brome peuvent être préparés par action du N-bromosuccinimide sur les dérivés correspondants de méthylbenzène en présence de peroxyde de dibenzoyle. On peut également préparer un bromure de benzyle à partir d'un alcool benzylique correspondant par action de l'acide bromhydrique en solution dans l'eau ou dans l'acide acétique. On peut aussi utiliser l'action du tribromure de phosphore sur un alcool benzylique correspondant pour préparer un composé de formule (VII) dans laquelle Hal représente un atome de brome.

Les composés de formule (VII) dans laquelle Hal représente un atome d'iode peuvent être préparés par action de l'iodure de sodium sur un composé de formule (VII) dans laquelle Hal représente un atome de chlore dans un solvant tel que l'acétone ou la butan-2-one.

Les composés de formule (VII) dans laquelle Hal représente un atome de chlore peuvent être préparés par action du chlorure de thionyle sur un alcool benzylique correspondant.

Les dérivés aminés de départ de formule (III) sont connus ou préparés par des méthodes connues, en particulier celles décrites dans EP-A-868 420. La (1S)-endo-1,3,3-triméthylbicyclo[2.2.1]heptan-2-ylamine est préparée selon J. Am. Chem. Soc., 1951, 73, 3360 ou selon J. Med. Chem., 1991, 34, 1003-1010. La bicyclo[3.2.1]octan-3-ylamine est préparée selon H. Maskill et al., J. Chem. Soc. Perkin Trans II, 1984, 1369.

Le composé de formule (I) obtenu par le procédé selon l'invention est isolé, sous forme de base libre ou de sel ou de solvate, selon les techniques conventionnelles.

Le composé de formule (I) peut être isolé sous forme d'un de ses sels, par exemple le chlorhydrate ou l'oxalate ; dans ce cas, la base libre peut être préparée par neutralisation dudit sel avec une base minérale ou organique, telle que l'hydroxyde de sodium ou d'ammonium, la triéthylamine ou un carbonate ou bicarbonate alcalin tel que le carbonate ou le bicarbonate de sodium ou de potassium, et transformée en un autre sel comme le méthanesulfonate, le fumarate ou le 2-naphtalènesulfonate.

Lorsque le composé de formule (I) est obtenu sous forme de base libre, la salification est effectuée par traitement avec l'acide choisi dans un solvant organique. Par traitement de la base libre, dissoute par exemple dans un éther tel que l'éther diéthylique ou dans l'acétone, avec une solution de l'acide dans le même solvant, on obtient le sel correspondant qui est isolé selon les techniques classiques.

Les composés de formule (I) possèdent une très bonne affinité *in vitro* pour les récepteurs aux cannabinoïdes CB₂, dans les conditions expérimentales décrites par Devane et al., Mol. Pharmacol., 1988, 34, 605-613.

Plus particulièrement, les composés de la présente invention, tels quels ou sous forme d'un de leurs sels pharmaceutiquement acceptables, sont des antagonistes puissants et sélectifs des récepteurs aux cannabinoïdes CB₂, ayant un Ki inférieur à 5.10⁻⁷ M. Ils sont au moms 10 fois plus actifs sur les récepteurs CB₂ que sur les récepteurs CB₁ et sont actifs par voie orale.

D'autre part, leur nature antagoniste a été démontrée par les résultats dans les modèles de l'inhibition de l'adénylate-cyclase induite par la forskoline comme décrit dans M. Rinaldi-Carmona et al., J. Pharm. Exp. Therap., 1998, 284, 644-650.

La toxicité des composés (I) est compatible avec leur utilisation en tant que médicament.

Selon un autre de ses aspects, la présente invention concerne l'utilisation des composés de formule (I), ou de l'un de leurs sels et solvats pharmaceutiquement acceptables, pour la préparation de médicaments destinés à traiter les maladies impliquant les récepteurs aux cannabinoïdes CB₂.

Les maladies pour le traitement desquelles les composés (I) et, éventuellement leurs sels pharmaceutiquement acceptables, peuvent être utilisés sont les pathologies impliquant les cellules du système immunitaire ou les désordres immunitaires, par exemple le SIDA, les maladies autoimmunes, les maladies associées aux transplantations d'organes, les maladies infectieuses, les maladies allergiques, les maladies du système gastrointestinal par exemple la maladie de Crohn, le syndrome du colon inflammatoire (inflammatory bowel disease) ; plus particulièrement on peut citer les maladies autoimmunes suivantes : lupus érythémateux disséminé, maladies du tissu conjonctif ou connectivites, syndrome de Sjögren's, spondylarthrite ankylosante, arthrite réactive, polyarthrite rhumatoïde, spondylarthrite indifférenciée, maladie de Behcet, anémies autoimmunes hémolytiques, sclérose en plaque, psoriasis. Les maladies allergiques à traiter peuvent être du type hypersensibilité immédiate ou retardé, asthme par exemple. De même les composés et leurs sels éventuels pharmaceutiquement acceptables peuvent être utilisés pour traiter les vascularites, les infections parasitaires, les infections virales, les infections bactériennes, l'amylose, les maladies affectant les lignées du système lymphohématopoïetique.

Les composés selon l'invention sont également utiles comme antiinflammatoire, comme antiartritique, comme analgésique, dans le traitement et la prévention des vertiges, de l'émésis et des nausées, en particulier les nausées induites par des agents anticancéreux, dans la traitement du diabète et dans le traitement des maladies oculaires, par exemple l'hypertension oculaire ou le glaucome.

De plus, les composés selon l'invention peuvent être utiles dans le traitement de certaines maladies du système nerveux central ou périphérique, par exemple l'épilepsie, les désordres psychotiques, la maladie d'Alzheimer, la maladie de Parkinson, la maladie de Tourette, la chorée de Huntington, ainsi que dans le traitement de certains cancers.

Ainsi, selon un autre de ses aspects, la présente invention concerne une méthode de traitement des maladies ci-dessus qui consiste à administrer à un patient en ayant besoin une quantité efficace d'un composé de formule (I) ou d'un de ses sels pharmaceutiquement acceptables.

Par ailleurs, les composés (I) selon l'invention, tels quels ou sous forme radiomarquée peuvent être utilisés comme outils pharmacologiques chez l'homme ou chez l'animal, pour la détection et le marquage des récepteurs périphériques CB₂ aux cannabinoïdes. Cela constitue un aspect ultérieure de la présente invention.

Les composés selon l'invention sont généralement administrés en unité de dosage.

Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I), un de ses sels pharmaceutiquement acceptables ou un de leurs solvates.
Les composés de formule (I) ci-dessus et leurs sels ou solvates pharmaceutiquement acceptables peuvent être utilisés à des doses journalières de 0,01 à 100 mg par kg de poids corporel du mammifère à traiter, de préférence à des doses journalières de 0,1 à 50 mg/kg. Chez l'être humain, la dose peut varier de préférence de 0,5 à 4000 mg par jour, plus particulièrement de 2 à 1000 mg par jour selon l'âge du sujet à traiter ou le type de traitement, à savoir prophylactique ou curatif. Bien que ces dosages soient des exemples de situation moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

Dans les compositions pharmaceutiques de la présente invention pour l'administration orale, sublinguale, inhalée, sous-cutanée, intramusculaire, intraveineuse, transdermique, locale ou rectale, le principe actif peut être administré sous forme unitaire d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les formes d'administration topique, les implants, les formes d'administration sous-cutanée, intramusculaire, intraveineuse, intranasale ou intra-oculaire et les formes d'administration rectale.

Dans les compositions pharmaceutiques de la présente invention, le principe actif est généralement formulé en unités de dosage contenant de 0,1 à 1000 mg, avantageusement de 0,5 à 500 mg, de préférence de 1 à 200 mg dudit principe actif par unité de dosage pour les administrations quotidiennes.

Lorsque l'on prépare une composition solide sous forme de comprimé, on peut ajouter au principe actif, micronisé ou non, un agent mouillant tel que le laurylsulfate de sodium et on mélange le tout avec un véhicule pharmaceutique tel que la silice, la gélatine, l'amidon, le lactose, le stéarate de magnésium, le talc, la gomme arabique ou analogues. On peut enrober les comprimés de saccharose, de divers polymères ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant tel qu'un glycol ou un ester de glycérol et en incorporant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir le principe actif en mélange avec des agents de dispersion, des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intra-oculaire, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol ou le polyéthylèneglycol.

Ainsi, pour préparer une solution aqueuse injectable par voie intraveineuse, on peut utiliser un cosolvant comme par exemple un alcool tel que l'éthanol ou un glycol tel que le polyéthylèneglycol ou le propylène glycol, et un tensioactif hydrophile tel que le Tween® 80. Pour préparer une solution huileuse injectable par voie intramusculaire, on peut solubiliser le principe actif par un triglycéride ou un ester de glycérol.

Pour l'administration locale on peut utiliser des crèmes, des pommades ou des gels.

Pour l'administration transdermique, on peut utiliser des patches sous forme multilaminée ou à réservoirs dans lesquels le principe actif peut être en solution alcoolique.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane ou tout autre gaz propulseur biologiquement compatible ; on peut également utiliser un système contenant le principe actif seul ou associé à un excipient, sous forme de poudre.

Le principe actif peut être formulé également sous forme de microcapsules ou microsphères, éventuellement avec un ou plusieurs supports ou additifs.

Le principe actif peut être également présenté sous forme de complexe avec une cyclodextrine, par exemple α-, β- ou γ-cyclodextrine, 2-hydroxypropyl-β-cyclodextrine ou méthyl-β-cyclodextrine.

Parmi les formes à libération prolongée utiles dans le cas de traitements chroniques, on peut utiliser les implants. Ceux-ci peuvent être préparés sous forme de suspension huileuse ou sous forme de suspension de microsphères dans un milieu isotonique.

### PREPARATIONS

### PREPARATION 1.1 Ester éthylique de l'acide 6-chloro-1,4-dihydroindeno[1,2-c]pyrazole-3-carboxylique.

### (VI) : X = CH₂, g₄ = Cl

### A) Ester éthylique du sel de lithium de l'acide (5-chloro-1-oxidoindan-2-yl)oxoacétique.

On prépare une solution de 4,42 g de LiHMDS à -60°C dans 140 ml d'Et₂O et on ajoute goutte à goutte une solution de 4,0 g de 5-chloroindan-1-one dans 10 ml d'Et₂O. On laisse 30 minutes sous agitation en laissant remonter la température jusqu'à -30°C puis on ajoute 3,6 ml d'oxalate d'éthyle. Après 18 heures sous agitation à TA, le précipité jaune obtenu est filtré, lavé à l'eau puis séché sous vide. On obtient 6,42 g du composé attendu.

### B) Ester éthylique de l'acide 6-chloro-1,4-dihydroindeno[1,2-c]pyrazole-3-carboxylique.

On ajoute 0,56 ml d'hydrate d'hydrazine à une solution refroidie au bain de glace contenant 3 g du composé obtenu à l'étape précédente dans 20 ml d'acide acétique. On chauffe à reflux pendant 18 heures puis on verse le mélange réactionnel dans 100 ml d'eau glacée. On filtre, lave à l'eau puis sèche sous vide pour obtenir 2,62 g du composé attendu, F = 190°C.

RMN : 1,25 ppm : t : 3H ; 3,70 ppm : s : 2H ; 4,25 ppm : q : 2H ; 7,25-7,65 ppm : m : 3H ; 13,80 ppm : s : 1H.

### PREPARATION 1.2

### Ester éthylique de l'acide 6-chloro-7-méthyl-1,4-dihydroindeno[1,2-c]pyrazole-3-carboxylique.

### (VI) : X = CH₂, g₃ = CH₃, g₄ = Cl

### A) 3-Chloro-1-(4-chloro-3-méthylphényl)propan-1-one.

On mélange 23,43 ml de 2-chlorotoluène et 26,6 g de chlorure de 3-chloropropionyle dans 25 ml de sulfure de carbone et on ajoute en 45 minutes 32 g de AlCl₃ dans 125 ml de sulfure de carbone. Après 3 heures sous agitation à TA, on évapore le solvant puis on ajoute 1 litre d'eau. On extrait le milieu réactionnel à l'éther puis au benzène, puis on lave la phase organique par une solution saturée de Na₂CO₃ puis par de l'eau. On sèche sur Na₂SO₄ puis on chromatographie le résidu sur colonne de silice en éluant par un mélange AcOEt/cyclohexane (5/95 ; v/v). On obtient 27,25 g du composé attendu.

RMN : : 2,4 ppm : s : 3H ; 3,6 ppm : t : 2H ; 3,9 ppm : t : 2H ; 7,6 ppm : m : 1H ; 7,8 ppm : m : 1H ; 8 ppm : m : 1H.

### B) 5-Chloro-6-méthylindanone.

A 30,67 g du composé préparé à l'étape précédente, on ajoute lentement et sous forte agitation 250 ml d'H₂SO₄ concentré. On chauffe à 90°C pendant une heure puis on verse sur de la glace. On extrait à l'éther puis on sèche la phase organique sur Na₂SO₄ et évapore à sec. Le résidu est chromatographié sur silice en éluant par un mélange AcOEt/cyclohexane (5/95 ; v/v). On obtient 1,8 g du composé attendu.

RMN : 2,3 ppm : s : 3H ; 2,5-2,6 ppm : m : 2H ; 2,9-3 ppm : m : 2H ; 7,5 ppm : s : 1H ; 7,6 ppm : s : 1H.

### C) Ester éthylique de l'acide 6-chloro-7-méthyl-1,4-dihydroindeno[1,2-c]pyrazole-3-carboxylique.

On procède ensuite comme décrit à la PREPARATION 1.1 aux étapes A et B pour préparer le composé attendu.

RMN : 1,2 ppm : t : 3H ; 2,3 ppm : s : 3H ; 3,6 ppm : s : 2H ; 4,2 ppm : q : 2H ; 7,4 ppm : s : 1H ; 7,5 ppm : s : 1H ; 13,7 ppm : s : 1H.

En procédant selon la PREPARATION 1.1 on prépare les composés décrits dans le TABLEAU 1.

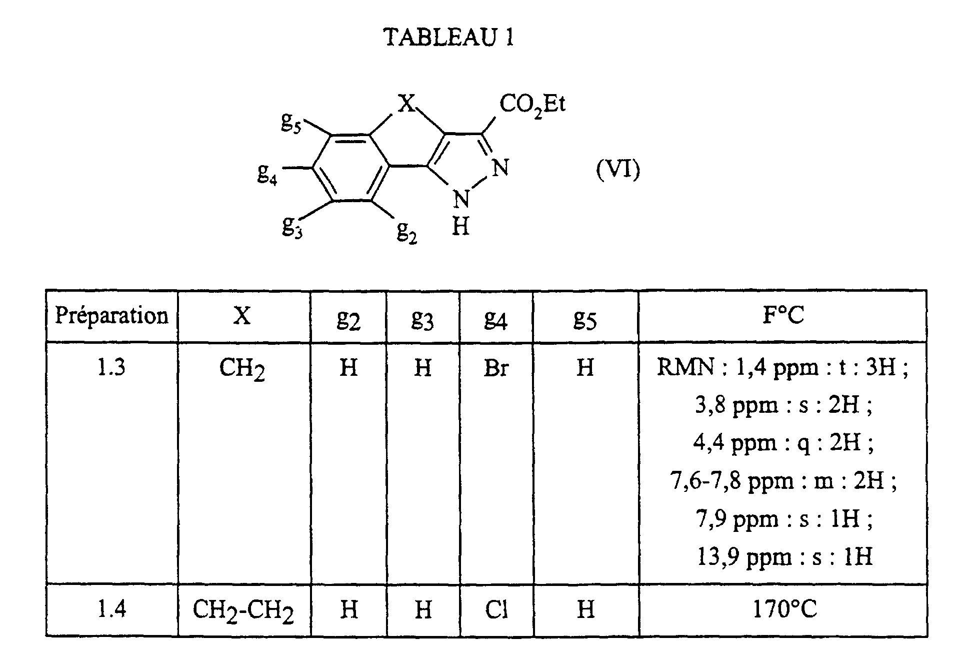

### PREPARATION 2.1

### Acide 1-(2,4-dichlorobenzyl)-6-chloro-1,4-dihydroindeno[1,2-c]pyrazole-3-carboxylique.

### II, X = CH₂; g₄ = w₂ = w₄ = Cl.

### A) Ester éthylique de l'acide 1-(2,4-dichlorobenzyl)-6-chloro-1,4-dihydroindeno [1,2-c]pyrazole-3-carboxylique.

On prépare une suspension de 2,48 g du composé obtenu à la PREPARATION 1 dans 50 ml de toluène et on ajoute en 3 fois 0,45 g d'hydrure de sodium à 60 % dans l'huile, puis on chauffe à 65°C pendant 1 heure. Après retour à TA, on ajoute 1,38 ml de chlorure de 2,4-dichlorobenzyle puis on chauffe à reflux pendant 44 heures. On ajoute 100 ml d'une solution de NH₄Cl saturé, on filtre le milieu, puis la phase organique est évaporée sous vide et le résidu est mélangé avec le précipité et trituré dans AcOEt. On filtre, lave par AcOEt puis sèche sous vide pour obtenir 3,00 g du composé attendu, F = 168°C.

### B) Acide 1-(2,4-dichlorobenzyl)-6-chloro-1,4-dihydroindeno[1,2-c]pyrazole-3-carboxylique.

On place 2,97 g du composé obtenu à l'étape précédente dans 60 ml de MeOH, on ajoute 1,01 g de KOH dans 10 ml d'eau puis on chauffe à reflux pendant 4 heures. Le milieu réactionnel est ensuite versé dans un mélange contenant 200 ml d'eau glacée et 20 ml d'HCl à 10 %. On filtre, lave à l'eau puis sèche sous vide pour obtenir 2,50 g du composé attendu, F > 260°C.

RMN : 3,75 ppm : s : 2H ; 5,80 ppm : s : 2H ; 7,05 ppm : d : 1H ; 7,30-7,80 ppm : m : 5H.

En procédant selon la PREPARATION 2.1, on prépare les composés décrits dans le TABLEAU ci-après.

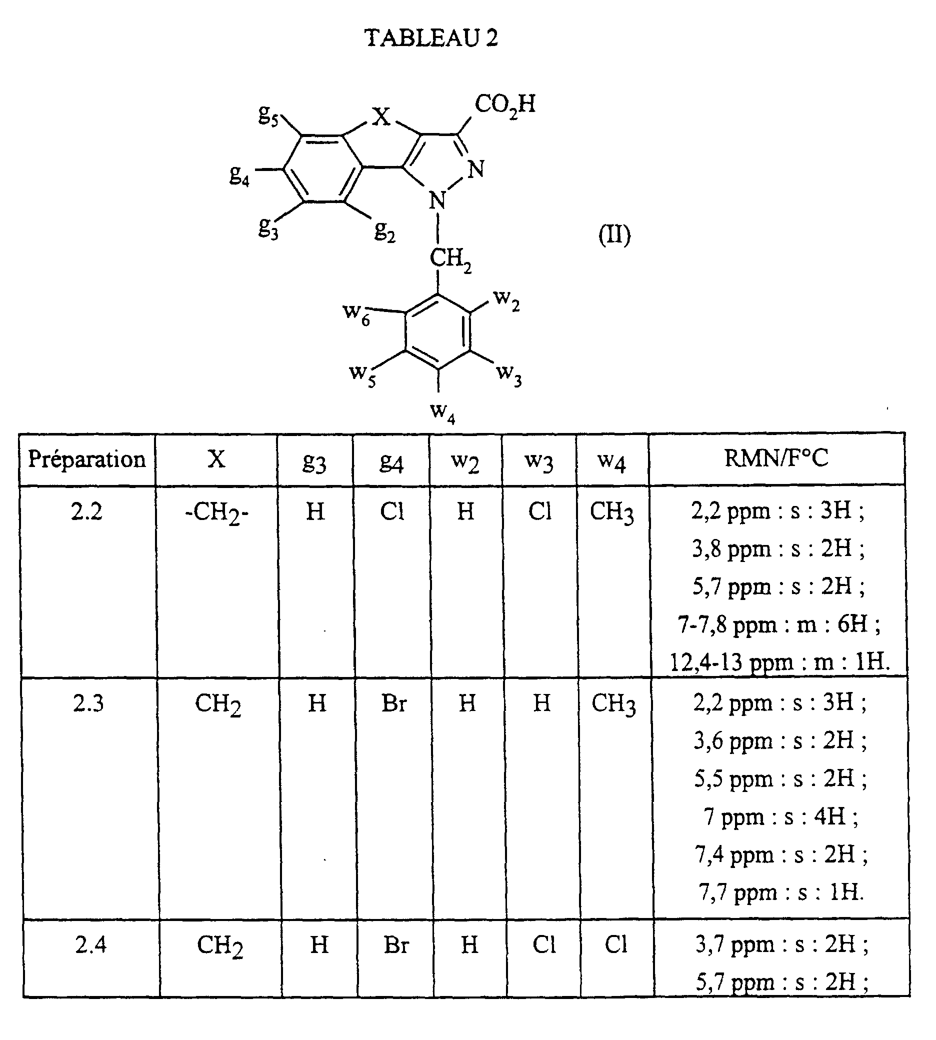

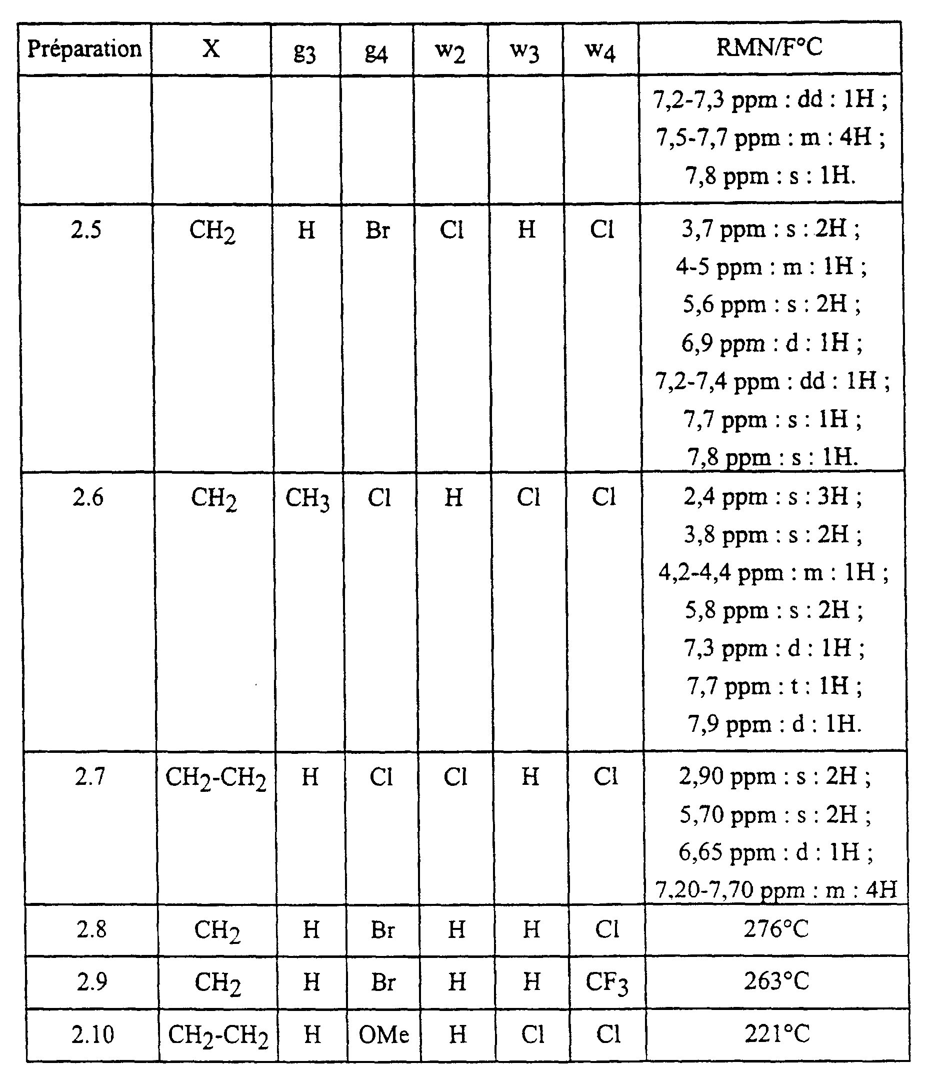

### PREPARATION 3

### 7,7-Diméthylbicyclo[4.1.0]hept-3-ylamine, chlorhydrate.

### A) 7,7-Diméthylbicyclo[4.1.0]heptan-3-one oxime.

On dissout 5 g de 7,7-diméthylbicyclo[4.1.0]hept-3-one dans 25 ml de MeOH et 18 ml de CHCl₃ et on ajoute 3,77 g d'hydroxylamine et 5,9 g d'acétate de sodium dissous dans 100 ml d'eau. On chauffe à reflux pendant 56 heures puis on refroidit. On extrait à l'éther puis on sèche la phase éthérée sur Na₂SO₄ et évapore à sec. L'huile obtenue (6,56 g) est utilisée telle quelle à l'étape suivante.

### B) 7,7-Diméthylbicyclo[4.1.0]hept-3-ylamine, chlorhydrate.

On dissout 6,5 g du produit obtenu à l'étape précédente dans 150 ml d'EtOH et on place dans une bombe à hydrogène. On ajoute 3 ml de CHCl₃ et 1,5 g de PtO₂ puis on laisse sous atmosphère d'H₂ à une pression de 7,8 bars pendant 72 heures. On filtre sur Célite® , évapore à sec, puis on reprend par de l'éther essore et sèche sous vide. On obtient 3,37 g du composé attendu.

RMN : 0,95 ppm : s : 3H ; 1,10 ppm : s : 3H ; 1,50-2,30 ppm : m : 8H ; 3,40-3,55 ppm : m : 1H ; 8,00 ppm : s : 2H.

### EXEMPLE 1

### N-[(1S)1,3,3-triméthylbicyclo[2.2.1]hept-2-endo-yl-6-chloro-1-(2,4-dichloro benzyl)-1,4-dihydroindeno[1,2-c]pyrazole-3-carboxamide.

### I, X = CH₂; g₄ = w₂ = w₄ = Cl;

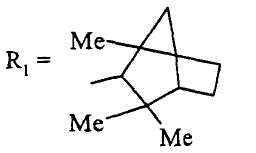

### A) Chlorure de l'acide 1-(2,4-dichlorobenzyl)-6-chloro-1,4-dihydroindeno[1,2-c] pyrazole-3-carboxylique.

On mélange 2,45 g du composé obtenu à la PREPARATION 2.1 dans 35 ml de toluène et 1,36 ml de SOCl₂ et on chauffe à reflux pendant 2 heures. On évapore le solvant sous vide puis on reprend le résidu par 30 ml de toluène et évapore à sec (2 fois). On obtient 2,59 g du composé attendu.

### B) N-[(1S)1,3,3-triméthylbicyclo[2.2.1]hept-2-endo-yl-6-chloro-1-(2,4-dichloro benzyl)-1,4-dihydroindeno[1,2-c]pyrazole-3-carboxamide.

On ajoute goutte à goutte une solution de 0,80 g du composé obtenu à l'étape précédente dans 20 ml de DCM sur une solution, refroidie à 0°C de 0,37 g de (1S)-endo-1,3,3-triméthylbicyclo[2.2.1]heptyl-2-ylamine et 0,55 ml de NEt₃ dans 20 ml de DCM. On laisse sous agitation à TA pendant 16 heures puis on verse le milieu réactionnel sur 100 ml d'eau glacée. On extrait au DCM, évapore le solvant, sèche sur MgSO₄ puis on chromatographie le résidu sur silice en éluant par un mélange AcOEt/toluène (1/9 ; v/v). Le composé obtenu cnstallise dans l'éther isopropylique. On obtient 0,46 g, F = 157°C.

RMN : 0,75 ppm : s : 3H ; 1,00-1,75 ppm : m : 13H ; 3,65 ppm : d : 1H ; 3,80 ppm : s : 2H ; 5,80 ppm : s : 2H ; 6,90-7,10 ppm : m : 2H ; 7,35-7,45 ppm : m : 2H ; 7,55-7,70 ppm : m : 3H.

En procédant selon l'EXEMPLE 1, on prépare les composés selon l'invention décrit dans le TABLEAU ci-après.

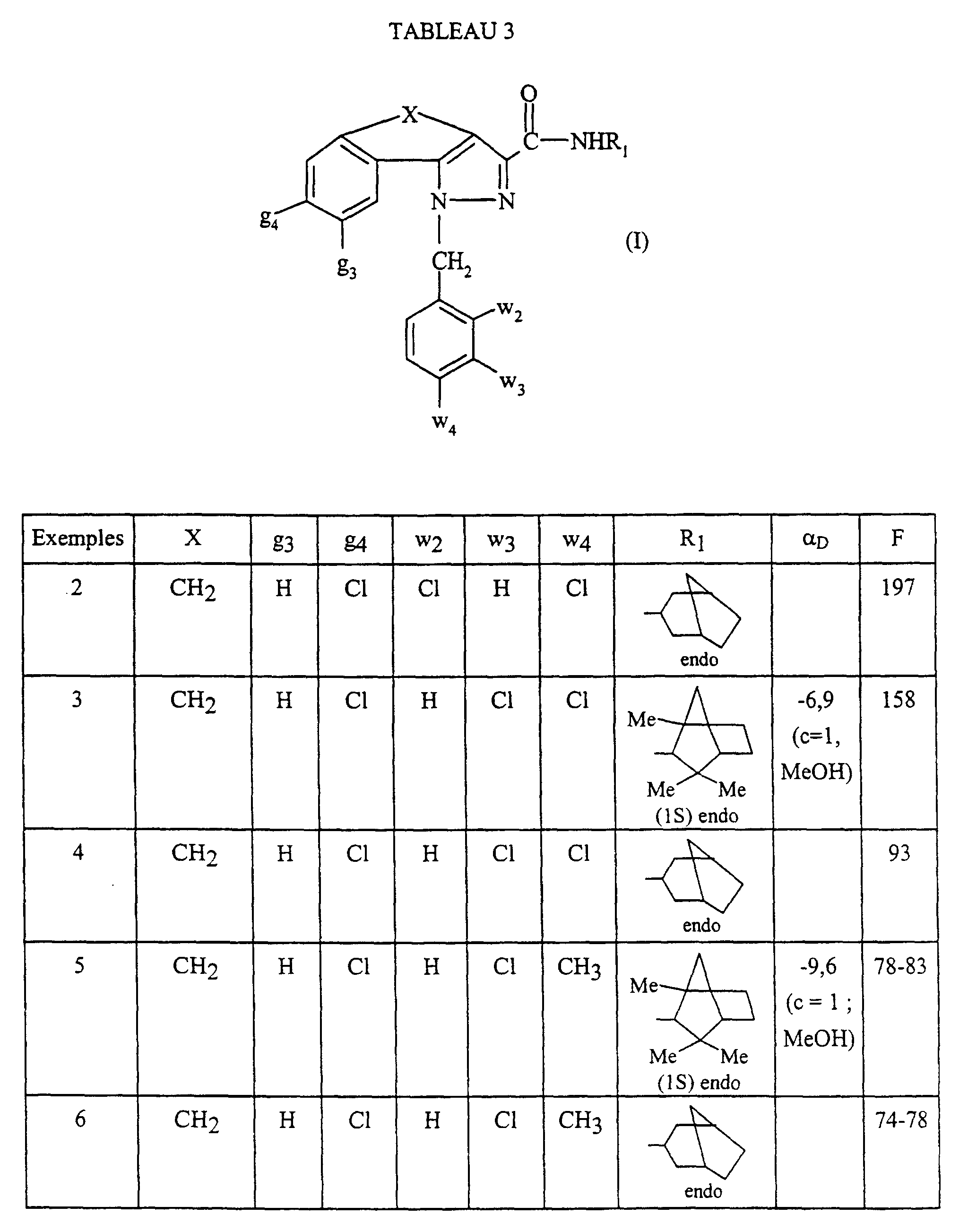

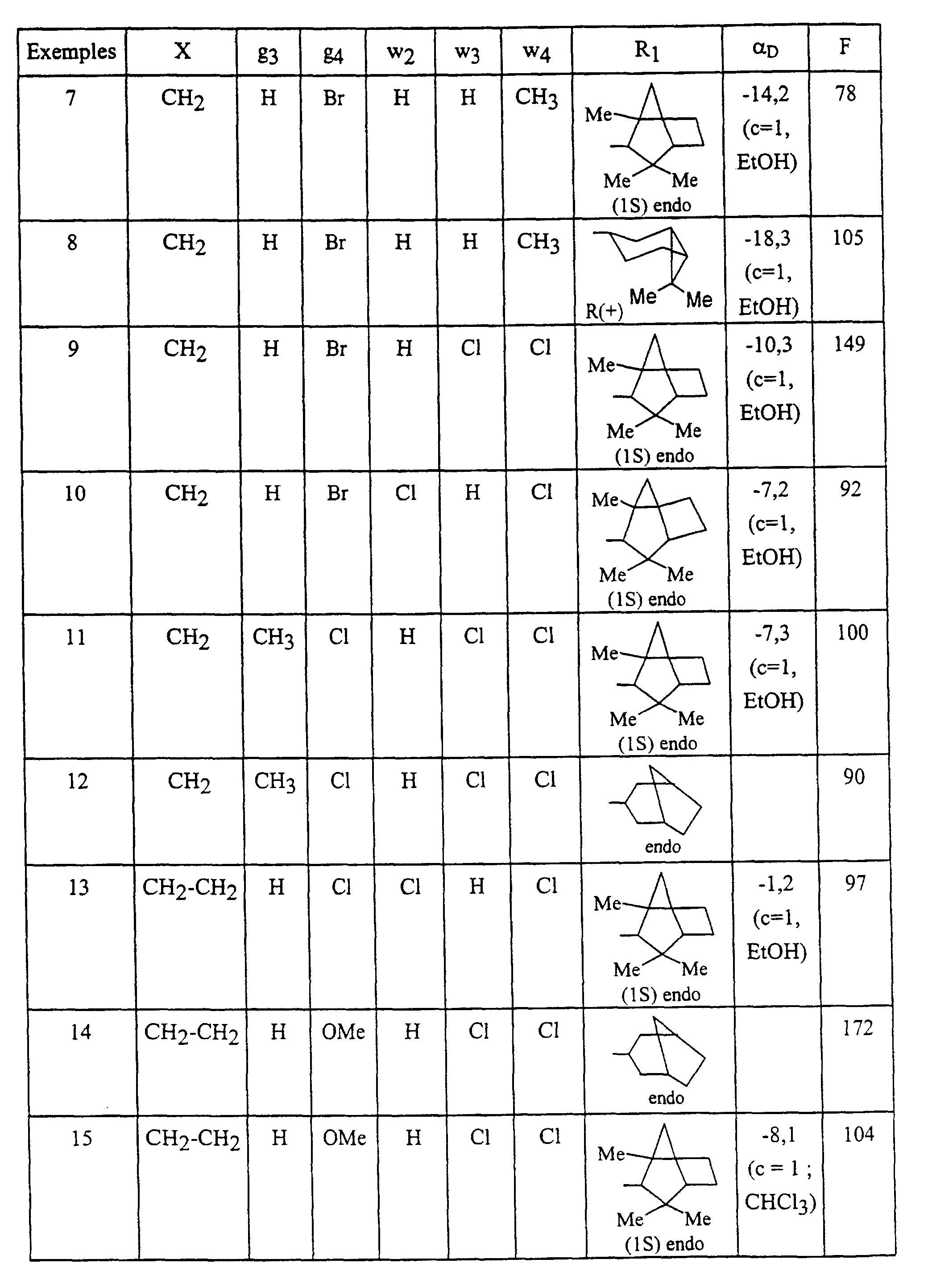

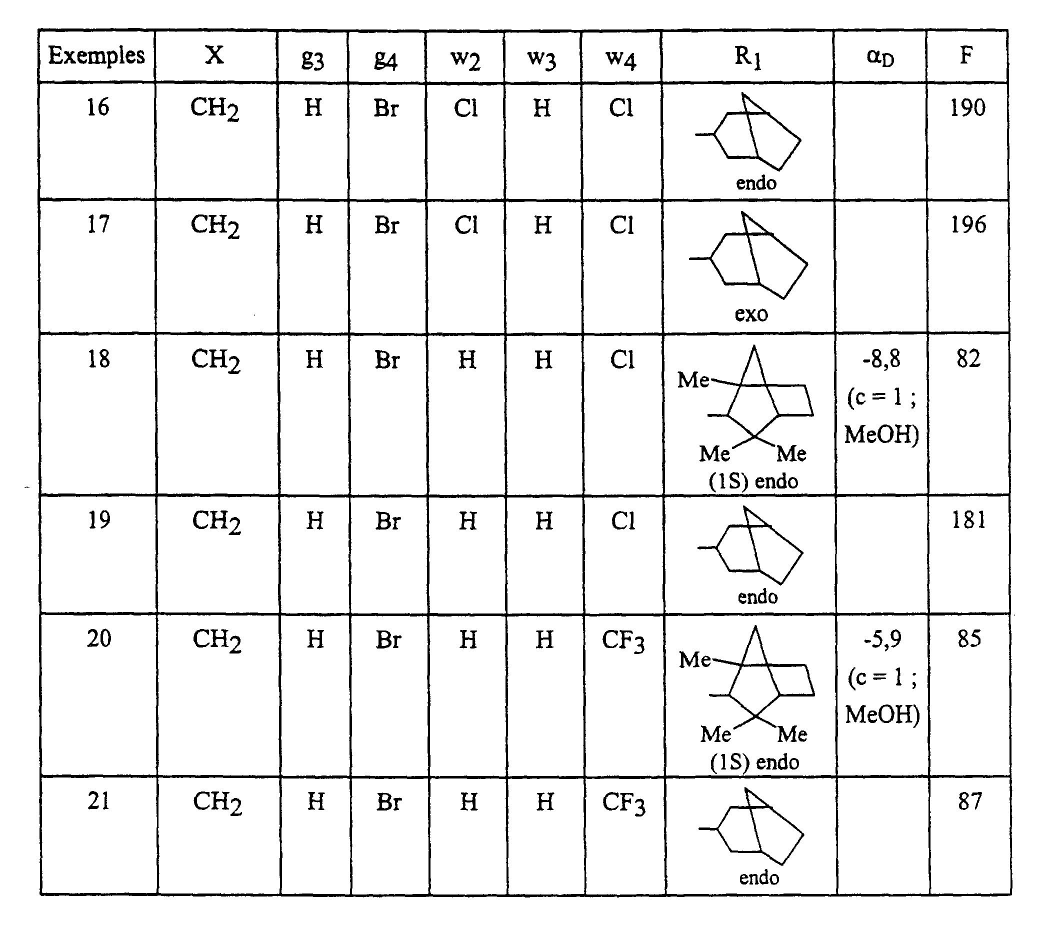

## Revendications

1. Un composé de formule : 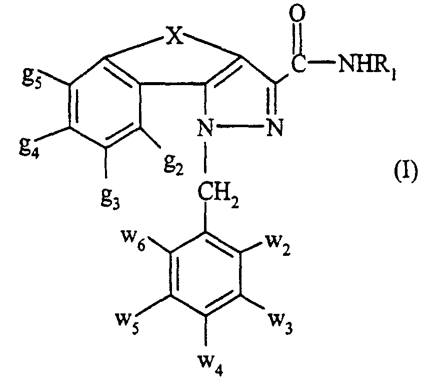 dans laquelle :
- X- représente un groupe -(CH₂)ₙ- ;
- n est égal à 1 ou 2 ;
- g₂, g₃, g₄, g₅, w₂, w₃, w₄, w₅, w₆ sont identiques ou différents et représentent chacun indépendamment l'hydrogène, un halogène, un trifluorométhyle, un (C₁-C₄)alkyle, un (C₁-C₄)alcoxy, un (C₁-C₄)alkylthio, un nitro ;
- R₁ représente un radical carbocyclique non aromatique en C₃-C₁₅ non substitué ou substitué une ou plusieurs fois par un (C₁-C₄)alkyle ;
ainsi que ses sels et ses solvats.

2. Un composé selon la revendication 1 dans lequel g₂, g₅, w₅, w₆ représentent l'hydrogène.

3. Un composé selon la revendication 2 dans lesquel w₂, w₃ et w₄ représentent le chlore ou un méthyle et g₃ et g₄ représente le chlore, le brome ou un méthyle.

4. Un composé selon l'une quelconque des revendications 1 à 3 de formule (I) dans laquelle R₁ représente un radical carbocyclique choisi parmi : le 1,3,3-triméthylbicyclo[2.2.1]hept-2-yle, le bicyclo[3.2.1]oct-3-yle, le 7,7-diméthylbicyclo[4.1.0]hept-3-yle.

5. Un composé selon la revendication 1 de formule (I) dans laquelle -X- représente -CH₂-.

6. Un procédé de préparation d'un composé selon la revendication 1 **caractérisé en ce que** l'on traite un dérivé fonctionnel d'un acide de formule : 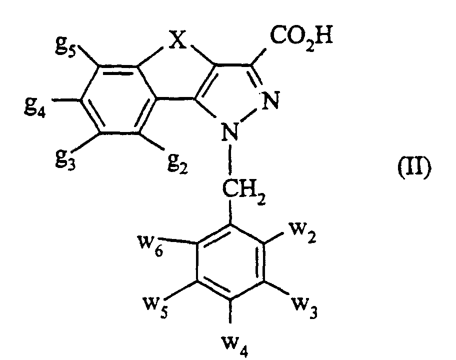 dans laquelle -X- et g₂, g₃, g₄, g₅, w₂, w₃, w₄, w₅, w₆ sont tels que définis à la revendication 1 pour (I), avec un composé de formule NH₂R₁ (III), dans laquelle R₁ est tel que défini à la revendication 1.

7. Un acide de formule : 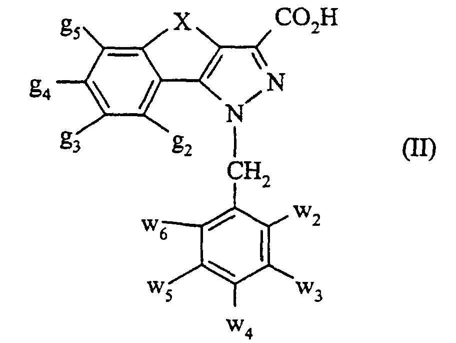 dans laquelle g₂, g₃, g₄, g₅, w₂, w₃, w₄, w₅, w₆ sont tels que définis à la revendication 1 ;
et ses dérivés fonctionnels.

8. Un composé selon la revendication 7 dans lequel les dérivés fonctionnels sont choisis parmi le chlorure d'acide, l'anhydride, un anhydride mixte, un ester alkylique en C₁-C₄ dans lequel l'alkyle est droit ou ramifié, un ester activé, tel que l'ester de *p*-nitrophényle ou l'acide libre activé, avec le N,N-dicyclohexyl carbodiimide ou avec l'hexafluorophosphate de benzotriazol-N-oxotris (diméthylamino)phosphonium.

9. Un composé selon la revendication 8 qui est le chlorure d'acide ou l'ester alkylique en C₁-C₄.

10. Composition pharmaceutique contenant un composé selon l'une quelconque des revendications 1 à 5.

11. Composition pharmaceutique selon la revendication 10 sous forme d'unité de dosage.

12. Utilisation d'un composé selon l'une quelconque des revendications 1 à 5 pour la préparation de médicaments destinés à traiter les maladies impliquant les récepteurs aux cannabinoïdes CB₂.

13. Utilisation selon la revendication 12 pour traiter les désordres immunitaires.

14. Utilisation selon la revendication 12 comme antiinflammatoire.

## Patentansprüche

1. Verbindung der Formel: 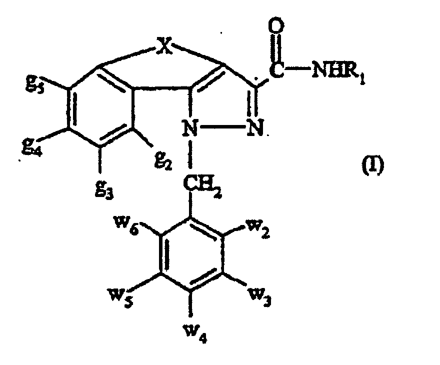 in der:
- -X- eine Gruppe -(CH₂)ₙ- darstellt;
- n 1 oder 2 bedeutet;
- g₂, g₃, g₄, g₅, w₂, w₃, w₄, w₅ und w₆ gleichartig oder verschieden sind und jeweils unabhängig voneinander Wasserstoff, Halogen, Trifluormethyl, (C₁-C₄)-Alkyl, (C₁-C₄)-Alkoxy, (C₁-C₄)-Alkylthio oder Nitro bedeuten;
- R₁ eine nichtaromatische, carbocyclische C₃-C₁₅-Gruppe bedeutet, die nicht substituiert ist oder ein oder mehrfach durch (C₁-C₄)-Alkyl substituiert ist;
sowie deren Salze und Solvate.

2. Verbindung nach Anspruch 1, worin g₂, g₅, w₅ und w₆ Wasserstoff bedeuten.

3. Verbindung nach Anspruch 2, worin w₂, w₃ und w₄ Chlor oder Methyl und g₃ und g₄ Chlor, Brom oder Methyl bedeuten.

4. Verbindung nach einem der Ansprüche 1 bis 3 der Formel (I), in der R₁ eine carbocyclische Gruppe bedeutet ausgewählt aus: 1,3,3-Trimethylbicyclo[2.2.1]-hept-2-yl, Bicyclo[3.2.1]oct-3-yl und 7,7-Dimethylbicyclo[4.1.0]hept-3-yl.

5. Verbindung nach Anspruch 1 der Formel (I), in der -X- -CH₂- bedeutet.

6. Verfahren zur Herstellung einer Verbindung nach Anspruch 1, **dadurch gekennzeichnet, daß** man ein funktionelles Derivat einer Säure der Formel: 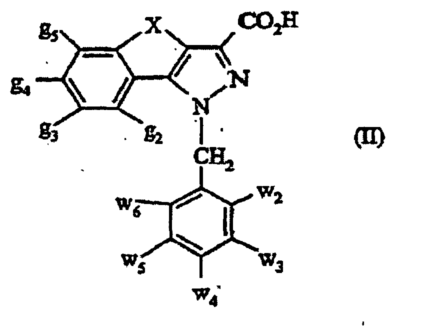 in der -X- und g₂, g₃, g₄, g₅, w₂, w₃, w₄, w₅ und w₆ die in Anspruch 1 für (I) angegebenen Bedeutungen besitzen, mit einer Verbindung der Formel NH₂R₁ (III) behandelt, in der R₁ die in Anspruch 1 angegebenen Bedeutungen besitzt.

7. Säure der Formel: 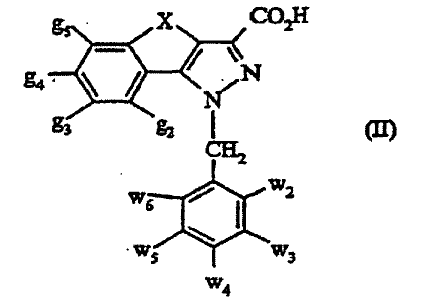 in der g₂, g₃, g₄, g₅, w₂, w₃, w₄, w₅ und w₆ die in Anspruch 1 angegebenen Bedeutungen besitzen,
und deren funktionelle Derivate.

8. Verbindung nach Anspruch 7, worin die funktionellen Derivate ausgewählt sind aus dem Säurechlorid, dem Anhydrid, einem gemischten Anhydrid, einem gemischten C₁-C₄-Alkylester, bei dem die Alkylgruppe geradkettig oder verzweigt ist, einem aktivierten Ester, wie dem *p*-Nitrophenylester, oder der mit N,N-Dicyclohexylcarbodiimid oder mit Benzotriazol-N-oxotris(dimethylamino)-phosphonium-hexafluorphosphat aktivierten freien Säure.

9. Verbindung nach Anspruch 8, welche das Säurechlorid oder der C₁-C₄-Alkylester der Säure ist.

10. Pharmazeutische Zubereitung enthaltend eine Verbindung nach einem der Ansprüche 1 bis 5.

11. Pharmazeutische Zubereitung nach Anspruch 10 in Dosiseinheitsform.

12. Verwendung einer Verbindung nach einem der Ansprüche 1 bis 5 für die Herstellung von Arzneimitteln zur Behandlung von Krankheiten, bei denen die Cannabinoid-Rezeptoren CB₂ eine Rolle spielen.

13. Verwendung nach Anspruch 12 für die Behandlung von Immunstörungen.

14. Verwendung nach Anspruch 12 als antiinflammatorisches Mittel.

## Claims

1. Compound of formula: 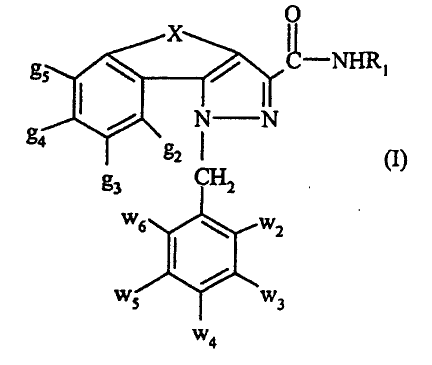 in which:
- X- represents a group -(CH₂)ₙ-;
- n is equal to 1 or 2;
- g₂, g₃, g₄, g₅, w₂, w₃, w₄, w₅, w₆ are identical or different and each independently represent hydrogen, a halogen, a trifluoromethyl, a (C₁-C₄)alkyl, a (C₁-C₄)alkoxy, a (C₁-C₄)alkylthio, a nitro;
- R₁ represents a nonaromatic C₃-C₁₅ carbocyclic radical which is unsubstituted or substituted one or several times with a (C₁-C₄)alkyl;
as well as its salts and its solvates.

2. Compound according to Claim 1, in which g₂, g₅, w₅, w₆ represent hydrogen.

3. Compound according to Claim 2, in which w₂, w₃ and w₄ represent chlorine or a methyl and g₃ and g₄ represents chlorine, bromine or a methyl.

4. Compound according to any one of Claims 1 to 3 of formula (I), in which R₁ represents a carbocyclic radical chosen from: 1,3,3-trimethylbicyclo[2.2.1]hept-2-yl, bicyclo[3.2.1]oct-3-yl, 7,7-dimethylbicyclo[4.1.0]hept-3-yl.

5. Compound according to Claim 1 of formula (I), in which -X- represents -CH₂-.

6. Method for preparing a compound according to Claim 1, **characterized in that** a functional derivative of an acid of formula: 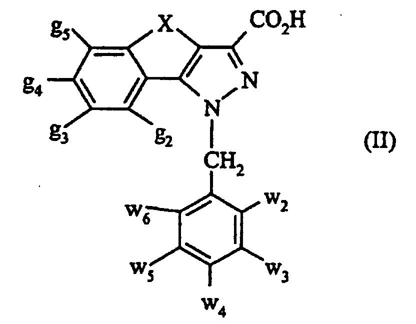 in which -X- and g₂, g₃, g₄, g₅, w₂, w₃, w₄, w₅, w₆ are as defined in Claim 1 for (I), is treated with a compound of formula NH₂R₁ (III), in which R₁ is as defined in Claim 1.

7. Acid of formula: 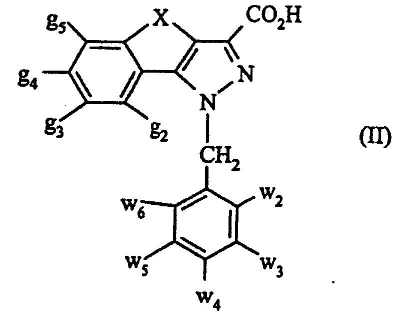 in which g₂, g₃, g₄, g₅, w₂, w₃, w₄, w₅, w₆ are as defined in Claim 1;
and its functional derivatives.

8. Compound according to Claim 7, in which the functional derivatives are chosen from the acid chloride, the anhydride, a mixed anhydride, a C₁-C₄ alkyl ester in which the alkyl is straight or branched, an activated ester, such as the *p*-nitrophenyl ester, or the free acid which is activated with N,N-dicyclohexylcarbodiimide or with benzotriazol-N-oxotris(dimethylamino)phosphonium hexafluorophosphate.

9. Compound according to Claim 8, which is the acid chloride or the C₁-C₄ alkyl ester.

10. Pharmaceutical composition containing a compound according to any one of Claims 1 to 5.

11. Pharmaceutical composition according to Claim 10, in dosage unit form.

12. Use of a compound according to any one of Claims 1 to 5 for the preparation of medicaments intended for treating diseases involving the cannabinoid CB₂ receptors.

13. Use according to Claim 12, for treating immune disorders.

14. Use according to Claim 12, as an antiinflammatory agent.
